# EUROPÄISCHE PATENTSCHRIFT

(11) **EP 2 303 828 B1**
(45) Veröffentlichungstag und Bekanntmachung des Hinweises auf die Patenterteilung: **30.07.2014**
(21) Anmeldenummer: 09769208.1
(22) Anmeldetag: 22.06.2009
(51) Int. Cl.: C07C 205/11, C07C 205/12

(54) **VERFAHREN ZUR HERSTELLUNG VON SUBSTITUIERTEN BIPHENYLEN**
METHOD FOR PRODUCING SUBSTITUTED BIPHENYLS
PROCÉDÉ DE FABRICATION DE BIPHÉNYLES SUBSTITUÉS

(30) Priorität: 25.06.2008 EP 08158963
(43) Veröffentlichungstag der Anmeldung: 06.04.2011
(60) Teilanmeldung: 13189221.8
(73) Patentinhaber: BASF SE, 67056 Ludwigshafen (DE)
(72) Erfinder: MAYWALD, Volker, 67071 Ludwigshafen (DE); SMIDT, Sebastian Peer, 68723 Oftersheim (DE); WISSEL-STOLL, Kathrin, 67056 Ludwigshafen (DE); SCHMIDT-LEITHOFF, Joachim, 68165 Mannheim (DE); ALTENHOFF, Ansgar Gereon, 69117 Heidelberg (DE); KEIL, Michael, 67251 Freinsheim (DE)
(86) Internationale Anmeldenummer: PCT/EP2009/057719
(87) Internationale Veröffentlichungsnummer: WO 2009/156359

(56) Entgegenhaltungen:
- WO-A-97/33846
- WO-A-2006/092429
- DATABASE BEILSTEIN [Online] BEILSTEIN INSTITUTE FOR ORGANIC CHEMISTRY, FRANKFURT-MAIN, DE; 1937, XP002507920 Database accession no. 3331999 (Beilstein registry number) & BELLAVITA ET AL.: GAZETTA CHIMICA ITALIANA, Bd. 67, 1937, Seiten 574-576,
- WANG SHEN: "Pallladium Catalyzed Coupling of Aryl Chlorides with Arylboronic Acids" TETRAHEDRON LETTERS, Bd. 38, Nr. 32, 1997, Seiten 5575-5578, XP002507918
- MITCHELL M B ET AL: "COUPLING OF HETEROARYL CHLORIDES WITH ARYLBORONIC ACIDS IN THE PRESENCE OF [1,4-BIS-(DIPHENYLPHOSPHINE)BUTANE]4 PALLADIUM(II) DICHLORIDE" TETRAHEDRON LETTERS, ELSEVIER, AMSTERDAM, Bd. 32, Nr. 20, 6. Mai 1991 (1991-05-06), Seiten 2273-2276, XP000195974 ISSN: 0040-4039
- CAI ET AL: "MCM-41-supported bidentate phosphine palladium(0) complex as an efficient catalyst for the heterogeneous Suzuki reaction" JOURNAL OF MOLECULAR CATALYSIS. A, CHEMICAL, ELSEVIER, AMSTERDAM, NL, Bd. 268, Nr. 1-2, 30. März 2007 (2007-03-30), Seiten 82-86, XP022008467 ISSN: 1381-1169
- WILLIAMS ET AL: "P-alkene bidentate ligands: an unusual ligand effect in Pd-catalysed Suzuki reactions" TETRAHEDRON, ELSEVIER SCIENCE PUBLISHERS, AMSTERDAM, NL, Bd. 63, Nr. 7, 18. Januar 2007 (2007-01-18), Seiten 1624-1629, XP005829478 ISSN: 0040-4020
- DATABASE CA [Online] CHEMICAL ABSTRACTS SERVICE, COLUMBUS, OHIO, US; 1929, HINKEL, LEONARD E. ET AL: "Conversion of hydroaromatic into aromatic compounds. III. 3,5-Dichloro-1-phenyl-.DELTA.2,4-cyclohexa diene and its behavior with chlorine" XP002577301 gefunden im STN Database accession no. 1929:7042 & HINKEL, LEONARD E. ET AL: "Conversion of hydroaromatic into aromatic compounds. III. 3,5-Dichloro-1-phenyl-.DELTA.2,4-cyclohexa diene and its behavior with chlorine" JOURNAL OF THE CHEMICAL SOCIETY 2786-91 CODEN: JCSOA9; ISSN: 0368-1769, 1928,
- DATABASE CA [Online] CHEMICAL ABSTRACTS SERVICE, COLUMBUS, OHIO, US; 1930, HINKEL, LEONARD E. ET AL: "Conversion of hydroaromatic into aromatic compounds. IV. The influence of the nitro group in nitrophenyldihydroresorcinols" XP002577302 gefunden im STN Database accession no. 1930:39440 & HINKEL, LEONARD E. ET AL: "Conversion of hydroaromatic into aromatic compounds. IV. The influence of the nitro group in nitrophenyldihydroresorcinols" JOURNAL OF THE CHEMICAL SOCIETY 1387-90 CODEN: JCSOA9; ISSN: 0368-1769, 1930,
- DATABASE CA [Online] CHEMICAL ABSTRACTS SERVICE, COLUMBUS, OHIO, US; 2007, LIU, CHUN ET AL: "Method for preparing biphenyl compound from chlorinated aromatic hydrocarbon and arylboronic acid via Suzuki cross-coupling reaction" XP002577303 gefunden im STN Database accession no. 2007:1149931 & CN 101 050 157 A (DALIAN UNIVERSITY OF TECHNOLOGY, PEOP. REP. CHINA) 10. Oktober 2007 (2007-10-10)

## Beschreibung

Die vorliegende Erfindung betrifft ein Verfahren zur Herstellung substituierter Biphenyle der Formel **I** 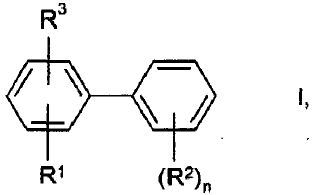 in der die Substituenten folgende Bedeutungen haben:
R¹ Nitro,
R² Cyano, Halogen, C₁-C₄-Halogenalkyl, C₁-C₄-Halogenalkoxy oder C₁-C₄-Halogenalkylthio,
n 0, 1, 2 oder 3, wobei im Falle von n = 2 oder 3 die Reste R² die gleichen oder verschiedene Bedeutungen haben können,
R³ Wasserstoff, Cyano oder Halogen,
dadurch gekennzeichnet, dass man ein Halogenbenzol der Formel II 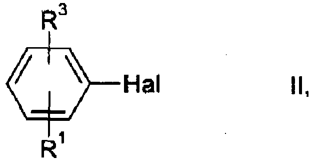
worin Hal für Chlor oder Brom steht und R¹ und R³ die oben angegebenen Bedeutungen haben,
in Gegenwart einer Base und eines Palladium-Katalysators, der aus Palladium und
einem zweizähnigen Phosphorliganden der Formel III 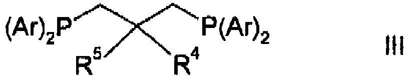 besteht, wobei Ar für Phenyl steht, das ein bis drei Substituenten tragen kann, ausgewählt aus der Gruppe bestehend aus Methyl, Methoxy, Fluor und Chlor, und R⁴ und R⁵ jeweils für C₁-C₈-Alkyl oder C₃-C₆₋Cycloalkyl stehen oder R⁴ und R⁵ zusammen eine 2- bis 7-gliedrige Brücke bilden, die gewünschtenfalls einen C₁-C₆-Alkylsubstituenten tragen kann,
in einem Lösungs- oder Verdünnungsmittel mit einer Phenylboronsäure IVa 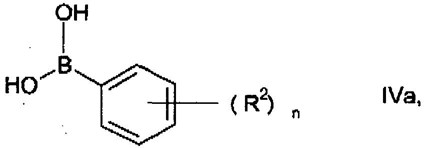 einer Diphenylborinsäure IVb 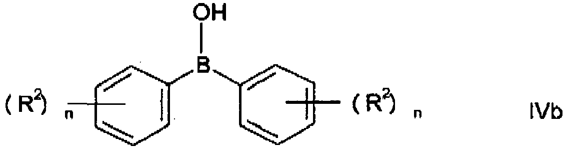 oder einem Gemisch aus IVa und IVb, worin R² und n jeweils die oben angegebenen Bedeutungen haben, umsetzt.

Palladium-katalysierte Kupplungen von Chloraromaten mit aromatischen Boronsäuren und Borinsäuren sind an sich bekannt. So wird zum Beispiel die Kupplung von 2-Nitrochlorbenzol mit halogensubstituierten aromatischen Boronsäuren zu den entsprechend substituierten Nitrobiphenylen in der WO 97/33846 beschrieben. Triphenylhosphin ist bei derartigen Kupplungsreaktionen der am häufigsten verwendete Ligand für das Palladium. Die Verwendung von Triphenylphosphin als Ligand birgt aber die Gefahr, dass die als Kupplungspartner verwendeten aromatischen Boron- und Borinsäuren in größerem Ausmaß protodeboroniert werden und die an dieser Position unsubstituierten aromatischen Verbindungen als unerwünschte Nebenprodukte entstehen. Dies gilt auch dann, wenn zur Verbesserung der Selektivität und Ausbeute Triphenylphosphin im deutlichen molaren Überschuss, bezogen auf die Palladiumquelle, eingesetzt wird.

In der Literatur wurde bisher nur selten über die Verwendung zweizähniger Bisphosphinliganden bei Kupplungsreaktionen berichtet. In der WO 98/16486 wird über die Verwendung lipophiler aliphatischer Bisphosphine in Reaktionen von einfachen Chloraromaten berichtet, jedoch ohne Beleg durch Beispiele.

Die DE-A 4340490 lehrt die Verwendung von 1,2-Bis-(dicyclohexylphosphanyl)-ethan und 1,2-Bis-(diethylphosphanyl)-ethan bei Kupplungsreaktionen. Mit elektronenreichen Chloraromaten wurden hier gute Ergebnisse erreicht, nicht jedoch mit Aromaten, die elektronenziehende Substituenten wie Fluor oder Trifluormethyl tragen.

Die Verwendung komplett aliphatisch substituierter Phosphane ist allerdings generell sehr eingeschränkt, da diese Phosphane sehr luftempfindlich und teilweise pyrophor sind.

S. Wang, Tetrahedron Letters 38(32), 5575-5578 (1997) offenbart die Suzuki-Kupplung von Nitrochlorbenzolen mit Phenylboronsäure, wobei der zweizähnige Ligand 1,3-Bis-diphenylphosphanyl-propan (dppp) verwendet wird.

Es wurde nun gefunden, dass die Palladium-katalysierte Reaktion von Chloraromaten mit halogensubstituierten aromatischen Boron- und Borinsäuren bei Verwendung des zweizähnigen Liganden dppp mit einer sehr viel schlechteren Ausbeute abläuft, als mit Triphenylphosphin (siehe Beispiel 2b im Vergleich zu 2a). Verwendet man aber einen zweizähnige Liganden, der durch weitere Substituenten in der Alkylkette leicht modifiziert ist, z. B. 1,3-Bis-diphenylphosphanyl-2,2-dimethylpropan oder 1,3-Bis-diphenylphosphanyl-2-ethyl-2-butyl-propan, so erhält man überraschenderweise die gewünschten Kupplungsprodukte in sehr guten Ausbeuten und zugleich nur sehr wenig protodeboronierte Nebenprodukte:

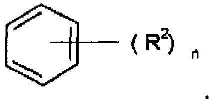

Ein weiterer Vorteil beim Einsatz dieser zuletzt genannten Liganden ist, dass sowohl die benötigte Menge der Palladiumquelle als auch die Menge des Liganden im Vergleich zur Fahrweise mit Triphenylphosphin deutlich reduziert werden kann. Da die eingesetzten Palladium-Verbindungen generell sehr teuer sind, ist diese Möglichkeit einer Verringerung des Palladium-Einsatzes von großem wirtschaftlichem Wert.

Der vorliegenden Erfindung lag daher die Aufgabe zugrunde, ein wirtschaftliches und im industriellen Maßstab technisch umsetzbares Verfahren zur regioselektiven Herstellung von substituierten Biphenylen bereitzustellen, welches mit einer verringerten Palladium-Katalysatorkonzentration arbeitet.

Das eingangs definierte Verfahren überwindet die dargelegten Nachteile des Standes der Technik.

Die nach dem vorliegenden Verfahren hergestellten substituierten Biphenyle I haben folgende bevorzugte Substituenten:
R² Cyano, Fluor, Chlor, Brom, Trifluormethyl, Trifluormethoxy oder Trifluormethylthio, besonders bevorzugt Fluor, Chlor oder Trifluormethylthio, ganz besonders bevorzugt Fluor oder Chlor;
n 2 oder 3, besonders bevorzugt 3;
R³ Wasserstoff oder Halogen, insbesondere Wasserstoff oder Fluor.
Ganz besonders bevorzugt ist die Herstellung von 3,4,5-Trifluor-2'-nitro-biphenyl.

Die Durchführung der sich anschließenden homogen katalysierten Suzuki Biaryl-Kreuzkupplung erfolgt bevorzugt nach folgendem Schema:

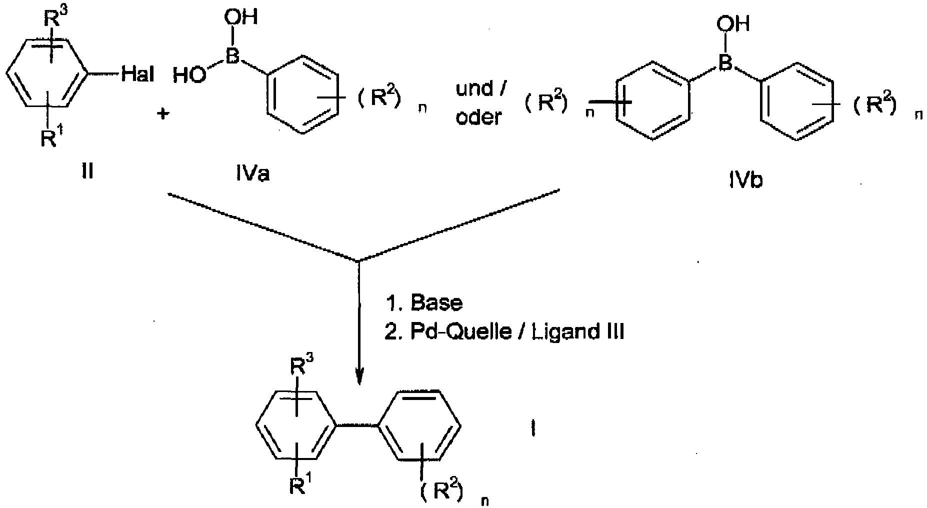

Dabei geht man bevorzugt von Phenylboronsäuren der Formel IVa oder Diphenylborlnsäuren der Formel IVb oder Gemischen aus diesen aus, in denen R² und n die oben angegebenen Bedeutungen haben.

Ganz besonders bevorzugt sind 3,4,5-Trifluorphenyl-boronsäure und Di(3,4,5-Trifluorphenyl)- borinsäure als Ausgangsverbindungen (IVa bzw. IVb).

Vorzugsweise geht man von Verbindungen (II) aus, welche eine einzige Nitrogruppe tragen, insbesondere 2-Nitrochlorbenzol.

Die Verbindung II wird, bezogen auf die Phenylboronsäure IVa oder die Diphenylborinsäure IVb, normalerweise etwa äquimolar, bevorzugt mit einem Überschuss, bis etwa 30-mol%, eingesetzt. Bei der Berechnung der molaren Verhältnisse und Überschüsse ist dabei zu berücksichtigen, dass die Diphenylborinsäure IVb, in reiner Form oder als Mischung mit IVa eingesetzt, zwei Phenylreste übertragen kann.

Als Base verwendet man vorzugsweise Alkalimetallhydroxide, Erdalkalimetallhydroxide, Alkalimetallcarbonate, Erdalkalimetallcarbonate, Alkalimetallhydrogencarbonate, Alkalimetallacetate, Erdalkalimetallacetate, Alkalimetallalkoholate und Erdalkalimetallalkoholate, im Gemisch und insbesondere einzeln.

Als Base besonders bevorzugt sind Alkalimetallhydroxide, Erdalkalimetallhydroxide, Alkalimetallcarbonate, Erdalkalimetallcarbonate und Alkalimetallhydrogencarbonate. Als Base insbesondere bevorzugt sind Alkalimetallhydroxide, z. B. Natriumhydroxid, Kaliumhydroxid und Lithiumhydroxid, sowie Alkalimetallcarbonate und Alkalimetallhydrogencarbonate, z. B. Lithiumcarbonat, Natriumcarbonat und Kaliumcarbonat.

Als Basen können aber auch organische Basen, z. B. tertiäre Amine, eingesetzt werden. Bevorzugt verwendet man z. B. Triethylamin oder Dimethylcyclohexylamin.

Die Base wird vorzugsweise mit einem Anteil von 100 bis 500 mol-%, besonders bevorzugt 150 bis 400 mol-%, bezogen auf die Phenylboronsäure IVa oder die Diphenylborinsäure IVb, eingesetzt.

Geeignete Palladium-Quellen sind zum Beispiel Palladium II-Salze wie Palladium-II-chlorid oder Palladium-II-acetat bzw. deren wässrige Lösungen, sowie Bisacetonitrilpalladium-II-chlorid oder Palladium-Komplexe mit Pd in der Oxidationsstufe 0.

Besonders bevorzugt wird Palladium-II-chlorid verwendet.

Geeignete in der Alkylkette substituierte, zweizähnige Phosphorliganden sowie deren Herstellung sind aus der älteren Anmeldung EP 08154184.9 bekannt.

Bevorzugt sind dabei 1,3-Bis-(diphenylphosphanyl)-propene III mit unsubstituierten Phenylringen, insbesondere diejenigen, bei denen R⁴ für C₁-C₆-Alkyl wie Methyl, Ethyl, 1-Methylethyl, n-Butyl, 1-Methylpropyl, 2- Methylpropyl, n-Pentyl, 1-Methylbutyl, 2-Methylbutyl, 3-Methylbutyl, n-Hexyl, 1-Methylpentyl, 2-Methylpentyl, 3-Methylpentyl und 4-Methylpentyl oder für Cyclopropyl und R⁵ für C₁-C₆-Alkyl wie Methyl, Ethyl, 1-Methylethyl, n-Butyl, 1-Methylpropyl, 2- Methylpropyl, n-Pentyl, 1-Methylbutyl, 2-Methylbutyl, 3-Methylbutyl, n-Hexyl, 1-Methylpentyl, 2-Methylpentyl, 3-Methylpentyl und 4-Methylpentyl stehen,
oder bei denen R⁴ + R⁵ gemeinsam eine Ethan-1,2-diyl-, Propan-1,3-diyl-, Butan-1,4-diyl- oder Pentan-1,5-diyl-Kette bedeuten.

Besonders bevorzugte Phosphorliganden III sind1,3-Bis-(diphenylphosphanyl)-2-methylpropan, 1,3-Bis-(diphenylphospanyl)-2,2-dimethylpropan, 1,3-Bis-(diphenylphosphanyl)-2-methyl-2-ethylpropan, 1,3-Bis-(diphenylphosphanyl)-2,2-dimethylpropan, 1,3-Bis-(diphenylphospanyl)-2-methyl-2-propylpropan, 1,3-Bis-(diphenylphosphanyl)-2-ethyl-2-propypropan, 1,3-Bis-(diphenylphosphanyl)-2,2-dipropylpropan, 1,3-Bis-(diphenylphosphanyl)-2-methyl-2-butylpropan, 1,3-Bis-(diphenylphosphanyl)-2-ethyl-2-butylpropan, 1,3-Bis-(diphenylphosphanyl)-2-propyl-2-butylpropan, 1,3-Bis-(diphenylphosphanyl)-2,2-dibutylpropan,
1,3-Bis-(diphenylphosphanyl)-2-methyl-2-cyclopropylpropan, 1,3-Bis-(diphenylphosphanyl)-2-methyl-2-cyclobutylpropan, 1,3-Bis-(diphenylphosphanyl)-2-methyl-2-cyclopentyl-propan, 1,3-Bis-(diphenylphosphanyl)-2-methyl-2-cyclohexylpropan, 1,1-Bis-(diphenylphosphanyl)-cyclopropan, 1,1-Bis-(diphenylphosphanyl)-cyclobutan, 1,1-Bis-(diphenylphosphanyl)-cyclopentan, 1,1-Bis-(diphenylphosphanyl)-cyclohexan, insbesondere 1,3-Bis-(diphenylphospanyl)-2,2-dimethylpropan und 1,3-Bis-(diphenylphosphanyl)-2-ethyl-2-butylpropan.

Die Reaktivität der Komplexliganden kann durch Zusatz eines quartären Ammoniumsalzes wieTetra-*n*-butylammoniumbromid (TBAB) gesteigert werden (vgl. z. B. D. Zim et al., Tetrahedron Lett. 2000, 41, 8199).

In der Regel werden 0,5 bis 5 Moläquivalente der vorstehend genannten Komplexliganden, insbesondere 1,3-Bis-diphenylphospanyl-2,2-dimethylpropan und 1,3-Bis-diphenylphosphanyl-2-ethyl-2-butylpropan mit einem Äquivalent des Palladium-IIsalzes kombiniert. Besonders bevorzugt ist der Einsatz von einem Moläquivalent Komplexligand, bezogen auf das Palladium-II-salz.

Die Palladiumquelle wird im erfindungsgemäßen Verfahren mit einem niedrigen Anteil von 0,001 bis 5,0 mol-%, vorzugsweise von 0,1 bis 1,0 mol-%, insbesondere von 0,1 bis 0,5 mol-%, bezogen auf die Verbindung IVa oder IVb eingesetzt.

Für das erfindungsgemäße Verfahren geeignete organische Lösungsmittel sind Ether wie Dimethoxyethan, Diethylenglykoldimethylether, Tetrahydrofuran, 2-Methyltetrahydrofuran, 3-Methyltetrahydrofuran, Dioxan, *tert*.-Butylmethylether und tert.-Butyl-ethylether, Kohlenwasserstoffe wie *n*-Hexan, *n*-Heptan, Heptan-Isomerengemische, Cyclohexan, Petrolether, Benzol, Toluol und Xylol, Alkohole wie Methanol, Ethanol, 1-Propanol, 2-Propanol, Ethylenglykol, 1-Butanol, 2-Butanol und *tert*.-Butanol, Ketone wie Aceton, Ethylmethylketon und *iso-*Butylmethylketon, Amide wie Dimethylformamid, Dimethylacetamid und *N-*Methylpyrrolidon, sowie Dimethylsulfoxid, jeweils einzeln oder in Mischung.

Bevorzugte Lösungsmittel sind Ether wie Dimethoxyethan, Tetrahydrofuran, 2-Methyltetrahydrofuran, 3-Methyltetrahydrofuran und Dioxan, Kohlenwasserstoffe wie Cyclohexan, Toluol und Xylol, Alkohole wie Ethanol, 1-propanol, 2-Propanol, 1-Butanol und *tert*.-Butanol, jeweils einzeln oder in Mischung.

In einer besonders bevorzugten Variante werden im erfindungsgemäßen Verfahren Wasser, ein oder mehrere in Wasser unlösliche und ein oder mehrere in Wasser lösliche Lösungsmittel eingesetzt, beispielsweise Mischungen aus Wasser und Dioxan oder Wasser und Tetrahydrofuran oder Wasser, Dioxan und Ethanol oder Wasser, Tetrahydrofuran und Methanol oder Wasser, Toluol und Tetrahydrofuran, vorzugsweise Wasser und Tetrahydrofuran oder Wasser, Tetrahydrofuran und Methanol. Bevorzugt wird die Reaktion in Wasser und Tetrahydrofuran durchgeführt.

Die Gesamtmenge an Lösungsmittel liegt normalerweise bei 3000 bis 100 und vorzugsweise bei 2000 bis 150 g pro Mol der Verbindung II.

Zweckmäßigerweise werden zur Durchführung des Verfahrens die Verbindung II, die Phenylboronsäure IVa bzw. die Diphenylborinsäure IVb, oder ein Gemisch aus beiden, die Base sowie die katalytische Menge der Palladium-Quelle in eine Mischung aus Wasser und einem oder mehreren inerten organischen Lösungsmitteln gegeben und bei einer Temperatur von 50°C bis 140°C, vorzugsweise 70°C bis 110°C, besonders bevorzugt 90°C bis 110°C, für einen Zeitraum von 1 bis 50, vorzugsweise 2 bis 24 Stunden, gerührt.

Je nach verwendetem Lösungsmittel und Temperatur stellt sich ein Druck von 1 bar bis 6 bar ein, bevorzugt 1 bar bis 4 bar.

Die Durchführung kann in üblichen für derartige Verfahren geeigneten Apparaturen erfolgen.

Nach beendeter Umsetzung kann der als Feststoff anfallender Palladium-Katalysator, beispielsweise durch Filtration, abgetrennt und das Rohprodukt vom Lösungsmittel bzw. den Lösungsmitteln befreit werden.

Anschließend kann nach dem Fachmann bekannten und dem jeweiligen Produkt angemessenen Methoden, z. B. durch Umkristallisation, Destillation, Sublimation, Zonenschmelzen, Schmelzkristallisation oder Chromatographie weiter aufgereinigt werden. Das erfindungsgemäße Verfahren liefert die Verbindungen I in sehr hohen bis zu quantitativen Ausbeuten bei sehr guter Reinheit.

Die Halogenbenzole II sind bekannt oder nach an sich bekannten Verfahren herstellbar.

Die Phenylboronsäuren IVa und die Diphenylborinsäuren IVb sind ebenfalls bekannt oder auf an sich bekannte Weise (vgl. z.B. die WO 2006/092429) herstellbar.

Die mit dem erfindungsgemäßen Verfahren erhältlichen substituierten Biphenyle I, beispielsweise 3,4-Difluor-2'-nitro-biphenyl, 2,4-Dichlor-2'-nitro-biphenyl, 3,4-Dichlor-2'-nitro-biphenyl und 3,4,5-Trifluor-2'-nitro-biphenyl, können auf an sich bekannte Weise mittels Hydrierung in die entsprechend substituierten Biphenyle mit R¹ = Amino übergeführt werden. Diese substituierten Biphenyle mit R¹ = Amino sind ihrerseits wichtige Zwischenprodukte für Pflanzenschutz-Wrkstoffe, beispielsweise fungizid wirksame Pyrazolcarboxamide V (vgl. z. B. EP-A 589301 oder WO 2006087343): 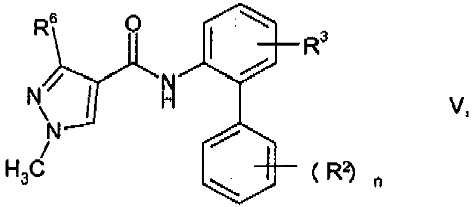 wobei R⁶ für Methyl oder Halogenmethyl wie Difluormethyl oder Trifluormethyl steht.

### Herstellungsbeispiel: Synthese von 3,4,5-Trifluor-2'-nitrobiphenyl

### a) Herstellung der 3,4,5-Trifluorphenyl-boronsäure

In einem mit Stickstoff oder Argon inertisierten Reaktor wurden zunächst 83,2 g (3,42 mol) Magnesiumspäne vorgelegt und anschließend 1646,2 g trockenes, unstabilisiertes Tetrahydrofuran zugegeben. 30 g (0,14 mol) 3,4,5-Trifluorbrombenzol wurden bei 25°C unter Rühren zugetropft und das Anspringen der Grignard-Reaktion abgewartet. Das Anspringen der Grignard-Reaktion machte sich durch eine spontane Temperaturerhöhung auf ca. 32°C bemerkbar. Anschließend wurden 571,9 g (2,71 mol) weiteres 3,4,5-Trifluorbrombenzol innerhalb von 5 Std. bei 25-35°C zudosiert. Zur Vervollständigung der Reaktion wurde 2 Std. bei 25-30°C nachgerührt. In einem 2. Reaktor wurde eine Lösung aus 328,0 g (3,16 mol) Trimethylborat und 452 g trockenem, unstabilisiertem Tetrahydrofuran vorgelegt und auf -5°C vorgekühlt. Danach wurde die Grignard-Lösung aus dem 1. Reaktor innerhalb von 2,5 Std. zudosiert. Das überschüssige Magnesium verblieb dabei im 1. Reaktor. Nach vollendeter Dosierung wurde noch 2 Std. bei 20-25°C nachgerührt. Zur Hydrolyse wurden anschließend 1326,1 g (2,76 mol) 7,6%ige Chlorwasserstoffsäure bei 25°C zudosiert, wonach man 1 Std. bei 25°C nachrührte. Das Gemisch wurde bis 50°C aufgeheizt und die Phasen getrennt. Danach wurde die organische Phase mit 603,9 g Wasser bei 50°C nachextrahiert und die Waschwasserphase erneut abgetrennt. Anschließend wurde durch Abdestillieren eines TetrahydrofuranlWasser-Gemisches die organische Phase aufkonzentriert. Man erhielt 1032,6 (82%) einer 40%igen Lösung der 3,4,5-Trifluorphenylboronsäure in Tetrahydrofuran, die direkt für die Folgeumsetzungen eingesetzt wurde.

### b) Herstellung von 3,4,5-Trifluor-2'-nitro-biphenyl durch Suzuki-Kupplung von 3,4,5-Trifluorphenylboronsäure mit 2-Nitro-chlorbenzol

In einem gut inertisierten Druckbehälter wurde eine Mischung aus 49,6 g (0,113 mol) einer 40%igen Lösung aus 3,4,5-Trifluorphenyl-boronsäure in Tetrahydrofuran aus Vorstufe a) mit 121,6 g (0,304 mol) einer 10%igen Natronlauge und 19,7 g (0,124 mol) 2-Nitrochlorbenzol vorgelegt. Dann wurde der jeweilige Ligand bei Raumtemperatur zugegeben, gerührt und zum Schluss das Palladium-II-chlorid zugegeben. Anschließend wurde das Reaktionsgemisch bis 105°C aufgeheizt. Dabei stellt sich ein Druck von ca. 3-4 bar ein. Nach etwa 12 Stunden Reaktionszeit wurde der Druckbehälter auf Normaldruck entspannt, auf 30°C abgekühlt und das Reaktionsgemisch abgelassen. Zur Aufarbeitung wurde das Reaktionsgemisch in tert.-Butylmethylether aufgenommen, die Phasen getrennt und die wässrige Phase zweimal mit tert.-Butylmethylether nachextrahiert. Die Solventien wurden bei reduziertem Druck vollständig abdestilliert, die Auswaage bestimmt und der Gehalt des rohen 3,4,5-Trifluor-2'-nitro-biphenyls mittels quantitativer HPLC analysiert. Gewünschtenfalls kann das rohe 3,4,5-Trifluor-2'-nitro-biphenyl weiter aufgereinigt werden, z.B. durch Kristallisation aus Isobutanol.

Nach Kristallisation aus Isobutanol erhält man das reine 3,4,5-Trifluor-2'-nitro-biphenyl mit einem Schmelzpunkt von 79°C.

| Bsp. | Ligand | mol% PdCl₂ | mol% Ligand | Verhältnis PdCl₂: Ligand | Ausbeute | mol% 3,4,5-Trifluorbenzol |
|---|---|---|---|---|---|---|
| b)-1 | Triphenylphosphan | 0,48 | 4,8 | 1:10 | 86% | 12 |
| b)-2 | 1,3-Bis-(diphenyl-phosphanyl)-propan | 0,26 | 0,26 | 1:1 | 9% | 29 |
| b)-3 | 1,3-Bis-(diphenyl-phosphanyl)-2,2-dimethylpropan | 0,26 | 0,26 | 1:1 | 97% | 2 |
| b)-4 | 1,3-Bis-(diphenyl-phosphanyl)-2-ethyl-2-butylpropan | 0,26 | 0,26 | 1:1 | 96% | 2 |

Die mol%-Angaben von PdCl₂ und Ligand sind jeweils auf die 3,4,5-Trifluorphenylboronsäure bezogen.

## Patentansprüche

1. Verfahren zur Herstellung substituierter Biphenyle der Formel I 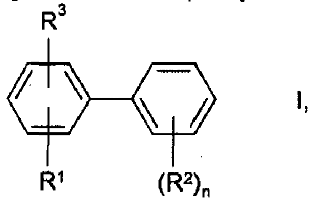 in der die Substituenten folgende Bedeutungen haben:
R¹ Nitro,
R² Cyano, Halogen, C₁-C₄-Halogenalkyl, C₁-C₄-Halogenalkoxy oder C₁-C₄-Halogenalkylthio,
n 0, 1, 2 oder 3, wobei im Falle von n = 2 oder 3 die Reste R² die gleichen oder verschiedene Bedeutungen haben können,
R³ Wasserstoff, Cyano oder Halogen,
**dadurch gekennzeichnet, dass** man ein Halogenbenzol der Formel II 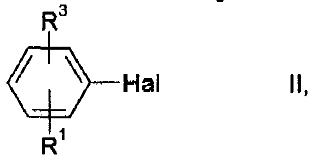
worin Hal für Chlor oder Brom steht und R¹ und R³ die oben angegebenen Bedeutungen haben,
in Gegenwart einer Base und eines Palladium-Katalysators, der aus Palladium und einem zweizähnigen Phosphorliganden der Formel III 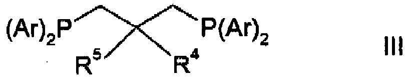
besteht, wobei Ar für Phenyl steht, das ein bis drei Substituenten tragen kann,
ausgewählt aus der Gruppe bestehend aus Methyl, Methoxy, Fluor und Chlor,
und R⁴ und R⁵ jeweils für C₁-C₈-Alkyl oder C₃₋C₆-Cycloalkyl stehen oder R⁴ und
R⁵ zusammen eine 2- bis 7-gliedrige Brücke bilden, die gewünschtenfalls einen C₁-C₆-Alkylsubstituenten tragen kann,
in einem Lösungs- oder Verdünnungsmittel mit einer Phenylboronsäure IVa 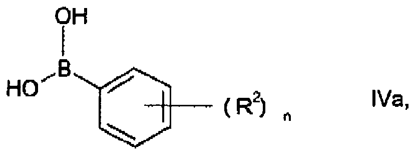 einer Diphenylborinsäure IVb 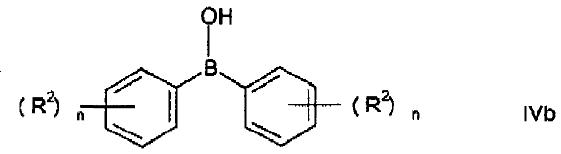
oder einem Gemisch aus IVa und IVb, worin R² und n jeweils die oben angegebenen Bedeutungen haben, umsetzt.

2. Verfahren nach Anspruch 1, **dadurch gekennzeichnet, dass** R² für Halogen, n für 2 oder 3 und R³ für Wasserstoff oder Halogen stehen.

3. Verfahren nach Anspruch 1, **dadurch gekennzeichnet, dass** R² für Fluor oder Chlor steht.

4. Verfahren nach Anspruch 1, **dadurch gekennzeichnet, dass** R¹ in ortho-Stellung zum Phenylring, der R² trägt, steht.

5. Verfahren nach Anspruch 1, **dadurch gekennzeichnet, dass** R¹ und R³ in para-Stellung zueinander stehen.

6. Verfahren nach Anspruch 1, **dadurch gekennzeichnet, dass** Biphenyle I ausgewählt aus der Gruppe bestehend aus 3,4-Difluor-2'-nitro-biphenyl, 2,4-Dichlor-2'-nitro-biphenyl, 3,4-Dichlor-2'-nitro-biphenyl und 3,4,5-Trifluor-2'-nitro-biphenyl hergestellt werden.

7. Verfahren nach Anspruch 1, **dadurch gekennzeichnet, dass** 3,4,5-Triluor-2'-nitro-biphenyl hergestellt wird.

8. Verfahren nach Anspruch 1, **dadurch gekennzeichnet, dass** Ar für Phenyl steht.

9. Verfahren nach Anspruch 1, **dadurch gekennzeichnet, dass** man die Umsetzung bei einer Temperatur von 50 bis 140°C durchführt.

10. Verfahren nach Anspruch 1, **dadurch gekennzeichnet, dass** man die Umsetzung in einem Gemisch aus Wasser und einem organischen Lösungsmittel durchführt.

11. Verfahren nach Anspruch 10, **dadurch gekennzeichnet, dass** man einen Ether als organisches Lösungsmittel verwendet.

12. Verfahren nach Anspruch 1, **dadurch gekennzeichnet, dass** man die Umsetzungen bei einem Druck von 1 bis 6 bar durchführt.

13. Verfahren nach Anspruch 1, **dadurch gekennzeichnet, dass** man die erhaltenen substituierten Biphenyle der Formel I anschließend auf an sich bekannte Weise zu Pyrazolcarboxamiden V 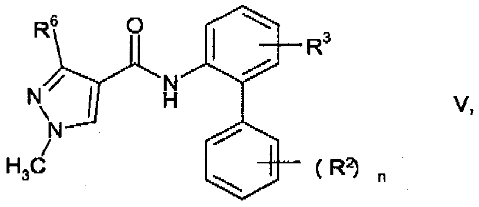 wobei n, R¹ und R² die in Anspruch 1 angegebenen Bedeutungen haben und
R⁶ für Methyl oder Halogenmethyl steht, umsetzt.

14. Verfahren nach Anspruch 13, **dadurch gekennzeichnet, dass** man die erhaltenen substituierten Biphenyle I ausgewählt aus der Gruppe bestehend aus 3,4-Difluor-2'-nitro-biphenyl, 2,4-Dichlor-2'-nitro-biphenyl, 3,4-Dichlor-2'-nitro-biphenyl und 3,4,5-Trifluor-2'-nitro-biphenyl umsetzt.

15. Verfahren nach Anspruch 13 oder 14, **dadurch gekennzeichnet, dass** man 3,4,5-Trifluor-2'-nitro-biphenyl umsetzt.

## Claims

1. A process for preparing substituted biphenyls of the formula I 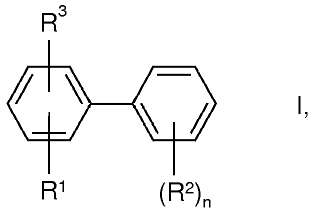 in which the substituents are each defined as follows:
R¹ is nitro,
R² is cyano, halogen, C₁-C₄-haloalkyl, C₁-C₄-haloalkoxy or C₁-C₄-haloalkylthio,
n is 0, 1, 2 or 3, where, in the case that n = 2 or 3, the R² radicals may have identical or different definitions,
R³ is hydrogen, cyano or halogen,
which comprises reacting a halobenzene of the formula II 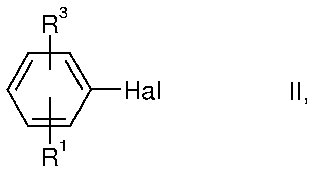 in which Hal is chlorine or bromine and R¹ and R³ are each as defined above,
in the presence of a base and of a palladium catalyst which consists of palladium and a bidentate phosphorus ligand of the formula III 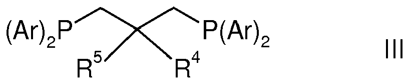
where Ar is phenyl which may bear from one to three substituents selected from the group consisting of methyl, methoxy, fluorine and chlorine, and R⁴ and R⁵ are each C₁-C₈-alkyl or C₃-C₆-cycloalkyl, or R⁴ and R⁵ together form a 2-to 7-membered bridge which may, if desired, bear a C₁-C₆-alkyl substituent,
in a solvent or diluent, with a phenylboronic acid Iva 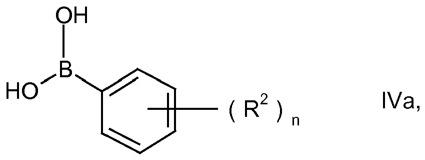
a diphenylborinic acid IVb 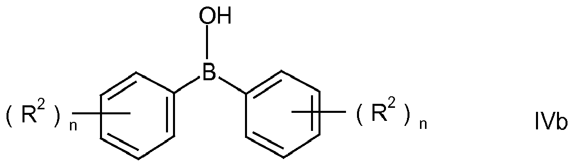
or a mixture of IVa and IVb, in which R² and n are each as defined as above.

2. The process according to claim 1, wherein R² is halogen, n is 2 or 3 and R³ is hydrogen or halogen.

3. The process according to claim 1, wherein R² is fluorine or chlorine.

4. The process according to claim 1, wherein R¹ is in the ortho position to the phenyl ring which bears R².

5. The process according to claim 1, wherein R¹ and R³ are in para positions to one another.

6. The process according to claim 1, wherein biphenyls I selected from the group consisting of 3,4-difluoro-2'-nitrobiphenyl, 2,4-dichloro-2'-nitrobiphenyl, 3,4-dichloro-2'-nitrobiphenyl and 3,4,5-trifluoro-2'-nitrobiphenyl are prepared.

7. The process according to claim 1, wherein 3,4,5-trifluoro-2'-nitrobiphenyl is prepared.

8. The process according to claim 1, wherein Ar is phenyl.

9. The process according to claim 1, wherein the reaction is performed at a temperature of from 50 to 140°C.

10. The process according to claim 1, wherein the reaction is performed in a mixture of water and an organic solvent.

11. The process according to claim 10, wherein an ether is used as the organic solvent.

12. The process according to claim 1, wherein the reactions are performed at a pressure of from 1 to 6 bar.

13. The process according to claim 1, wherein the resulting substituted biphenyls of the formula I are subsequently converted in a manner known per se to pyrazolecarboxamides V 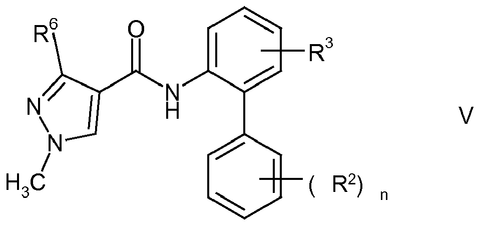 where n, R² and R³ are as defined in claim 1 and R⁶ is methyl or halomethyl.

14. The process according to claim 13, wherein the resulting substituted biphenyls I which are selected from the group consisting of 3,4-difluoro-2'-nitrobiphenyl, 2,4-dichloro-2'-nitro-biphenyl, 3,4-dichloro-2'-nitrobiphenyl and 3,4,5-trifluoro-2'-nitrobiphenyl, are converted.

15. The process according to claim 13 or 14, wherein 3,4,5-trifluoro-2'-nitrobiphenyl is converted.

## Revendications

1. Procédé pour la préparation de biphényles substitués de formule (I) 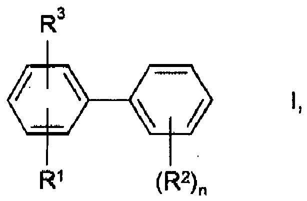 dans laquelle les substituants ont les significations suivantes :
R¹ nitro,
R² cyano, halogène, C₁-C₄-halogénoalkyle, C₁-C₄-halogénoalcoxy ou C₁-C₄-halogénoalkylthio,
n 0, 1, 2 ou 3, où, dans le cas de n = 2 ou 3, les radicaux R² peuvent présenter des significations identiques ou différentes,
R³ hydrogène, cyano ou halogène,
**caractérisé en ce qu'**on transforme un halogénobenzène
de formule II 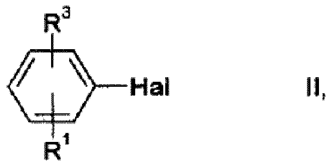
dans laquelle Hal représente chlore ou brome et R¹ et R³ présentent les significations indiquées ci-dessus,
en présence d'une base et d'un catalyseur de palladium,
qui est constitué par du palladium et un ligand phosphoré bidentate de formule III 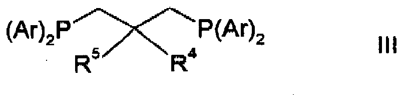
dans laquelle Ar représente phényle, qui peut porter un à trois substituants, choisis dans le groupe constitué par méthyle, méthoxy, fluor et chlore, et R⁴ et R⁵ représentent à chaque fois C₁-C₈-alkyle ou C₃-C₆-cycloalkyle ou R⁴ et R⁵ forment, ensemble, un pont de 2 à 7 chaînons, qui peut éventuellement porter un substituant C₁-C₆-alkyle,
dans un solvant ou un diluant, avec un acide
phénylboronique IVa 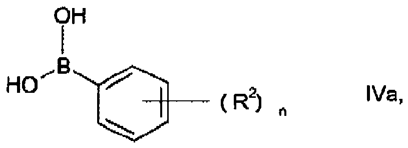
un acide diphénylborinique IVb 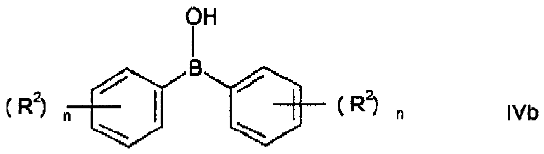
ou un mélange de IVa et IVb, dans lesquels R² et n présentent à chaque fois les significations susmentionnées.

2. Procédé selon la revendication 1, **caractérisé en ce que** R² représente halogène, n vaut 2 ou 3 et R³ représente hydrogène ou halogène.

3. Procédé selon la revendication 1, **caractérisé en ce que** R² représente fluor ou chlore.

4. Procédé selon la revendication 1, **caractérisé en ce que** R¹ se trouve en position ortho par rapport au cycle phényle qui porte R².

5. Procédé selon la revendication 1, **caractérisé en ce que** R¹ et R³ se trouvent en position para l'un par rapport à l'autre.

6. Procédé selon la revendication 1, **caractérisé en ce qu'**on prépare des biphényles I choisis dans le groupe constitué par le 3,4-difluoro-2'-nitro-biphényle, le 2,4-dichloro-2'-nitro-biphényle, le 3,4-dichloro-2'-nitro-biphényle et le 3,4,5-trifluoro-2'-nitro-biphényle.

7. Procédé selon la revendication 1, **caractérisé en ce qu'**on prépare du 3,4,5-trifluoro-2'-nitro-biphényle.

8. Procédé selon la revendication 1, **caractérisé en ce que** Ar représente phényle.

9. Procédé selon la revendication 1, **caractérisé en ce qu'**on réalise la transformation à une température de 50 à 140°C.

10. Procédé selon la revendication 1, **caractérisé en ce qu'**on réalise la transformation dans un mélange d'eau et d'un solvant organique.

11. Procédé selon la revendication 10, **caractérisé en ce qu'**on utilise un éther comme solvant organique.

12. Procédé selon la revendication 1, **caractérisé en ce qu'**on réalise les transformations à une pression de 1 à 6 bars.

13. Procédé selon la revendication 1, **caractérisé en ce qu'**on transforme ensuite les biphényles substitués de formule I obtenus de manière connue en soi en pyrazolecarboxamides V 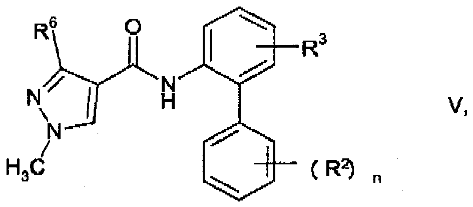 où n, R² et R³ présentent les significations indiquées dans la revendication 1 et R⁶ représente méthyle ou halogénométhyle.

14. Procédé selon la revendication 13, **caractérisé en ce qu'**on transforme les biphényles substitués I obtenus, choisis dans le groupe constitué par le 3,4-difluoro-2'-nitro-biphényle, le 2,4-dichloro-2'-nitro-biphényle, le 3,4-dichloro-2'-nitro-biphényle et le 3,4,5-trifluoro-2'-nitro-biphényle.

15. Procédé selon la revendication 13 ou 14, **caractérisé en ce qu'**on transforme du 3,4,5-trifluoro-2'-nitro-biphényle.
